# EUROPEAN PATENT APPLICATION

(11) **EP 4 796 556 A1**
(43) Date of publication of application: **26.08.2026**
(21) Application number: 24878955.4
(22) Date of filing: 14.10.2024
(51) Int. Cl.: C07K 1/107, C07K 1/04, C07K 5/103

(54) **SOLID-PHASE SIMPLE SYNTHESIS METHOD FOR PEPTIDOL**

(30) Priority: 16.10.2023 US 202363590468 P
(71) Applicant: Kaohsiung Medical University, Sanmin District Kaohsiung, Taiwan 80708 (CN)
(72) Inventor: KAO, Chai-Lin, New Taipei City Taiwan 234007 (CN); CHEN, Hui-Ting, Kaohsiung City Taiwan 813347 (CN); CHEN, Szu-Hsuan, Taoyuan City Taiwan 330058 (CN)
(74) Representative: Lang, Christian
(86) International application number: PCT/CN2024/124690
(87) International publication number: WO 2025/082311

(57) **Abstract**

A 3,4 diaminobenzoic acid (Dbz) group on a resin is activated by means of isoamyl nitrite to form benzotriazole (Bt), and a reduction reaction is then performed by using sodium borohydride (NaBH₄) as a reducing agent. A peptidol can be obtained by means of a rapid reaction and only a simple filtration method, and the generation of by-products accompanying with the reaction can be reduced by changing conditions.

## Description

### FIELD OF THE INVENTION

The present invention relates to a solid-phase synthesis method for preparing peptidol and derivatives thereof.

### BACKGROUND OF THE INVENTION

Peptides play many roles in living organisms and can function as hormones, neurotransmitters, growth factors, signaling transduction substances, and antibodies. Compared with proteins and antibodies, peptides are easier to synthesize and exhibit target specificity and selectivity, making them have great development potential in drug development and materials applications. Due to their virtually unlimited structural variations and their ability to allow modification of various functional groups, peptides can exhibit a wide range of biological activities, when compared to steroids, which have more limited variations in structure and functional group, and fatty acids, which, though they have structural flexibility, do not allow modification of functional groups.

After reduction at the C-terminus, the originally negatively charged carboxylic acid functional group at the C-terminus is altered, and the resulting peptidols become hydrophobic and aggregated because the repulsion force caused by negative charges is removed when the negative charge of the carboxylic acid at physiological pH is neutralized. Thereby altering the binding between the peptide and receptors. Through this subtle structural alteration, the peptide's folding, binding conditions with target sites, and pharmacokinetic properties are significantly altered. For example, Enkephalin, an endogenous opioid pentapeptide responsible for neurotransmission and pain regulation, exhibits a 30-fold increase in binding affinity to rat brain membranes when its C-terminus is modified to a hydroxyl group, exhibiting a significantly prolonged analgesic activity.

A chemically synthesized peptide typically possesses a carboxylic acid or amide group at the C-terminus. However, peptides with other types of C-terminal modifications are frequently found in natural products. These peptides have distinct biological activities, which demonstrate their potential as therapeutic agents. Among the current chemical synthetic methods for peptidols, general, convenient, and rapid methods remain scarce. Therefore, further development of chemical synthesis strategies for peptidols remains necessary. Currently, most methods for preparing peptidols involve anchoring amino alcohols to a resin for synthesis. An additional liquid-phase step is required to convert the amino acids into amino alcohols. As a result, modification of the linker is required, leading to long reaction times for coupling and cleaving the amino alcohol and exposing the synthesized peptide to strong acidic or basic conditions for extended periods, increasing the risk of racemization.

In solid-phase peptide synthesis (SPPS), a resin is used as a solid support, which is blocked in a container by using a frit. First, a deprotection reagent is used to remove protective groups from the resin-bound backbone, and the excess reagent is removed by washing. The amino acid to be synthesized is then dissolved in solvents with a coupling reagent and coupled to the resin. After the reaction, only simple washing is required to remove excess reagents and by-products, thereby improving the yield and addressing the efficiency limitations associated with liquid-phase synthesis. Its convenience and high efficiency make this method particularly advantageous for subsequent chemical synthesis of peptides. In solid-phase peptide synthesis, resin is used as a solid support. After being connected to a linker, the resin is coupled to the C-terminus of the amino acid, and then the N-terminus is exposed for subsequent reactions.

After coupling the amino acid to the resin, further modifications are performed. This approach is applicable to various C-terminal modifications of peptides. For example, starting with the Rink amide resin, a peptide can be cleaved with acid after completion of the synthesis to obtain a peptide with a C-terminal amide. Through the functional group attachment point provided by the linker, the desired functional group is introduced onto the peptide, modifying its C-terminus accordingly.

The difficulty in performing C-terminal modifications in solid-phase peptide synthesis arises from requiring C-terminus to be anchored on a resin. Instead, by connecting to the resin through side-chain functional groups or the N-terminus, the C-terminus can be exposed to alleviate the difficulties in C-terminal modifications. In 1971, the Kenner team proposed a safety-catch linker method, in which the C-terminus is connected to the resin. Prior to activation, the linker remains stable and unreactive during the solid-phase peptide synthesis process. After being activated, the linker can form a leaving group, allowing the C-terminus of the peptide to be modified with the target functional group through nucleophilic substitution reactions, while simultaneously cleaving the peptide from the solid phase.

In peptidol synthesis, the amino alcohol condensation method is a method that has been seen the most, indicating that when a peptidol is synthesized on a solid phase, efforts have primarily focused on developing new resin linkers that can be connected with a hydroxyl group. However, the coupling of the resin linker to the hydroxyl group often involves extended coupling time and low resin loading efficiency, and extended coupling time requires the amino acids to remain in a basic environment for a long period of time, increasing the risk of peptide racemization. In addition, amino alcohol synthesis also poses a significant challenge. Methods developed in the nucleophilic substitution method are less extensive than those in the amino alcohol condensation method. Only a few studies focus on solid-phase C-terminal modifications with reducing agents. However, the yields of these methods are generally not desirable, and the reduction time is long or require microwave to provide reaction energy. Furthermore, there is also a risk of racemization. The only method with a relatively short reaction time, however, suffers from severe hydrolysis of synthesized peptides. However, these methods can be applied to common amino acids because the amino acids are not required to be pre-modified into amino alcohols. Finally, non-carboxyl connected resin methods provide solid-phase connecting points other than the C-terminus, thereby reducing the difficulty in performing solid-phase C-terminal modifications. To date, no studies has reported a complete method for synthesizing peptidols. Therefore, there is indeed a need for an effective synthetic approach to overcome the challenges associated with peptidol synthesis.

### DETAILED DESCRIPTION OF THE INVENTION

Other features and advantages of the present invention are disclosed in the following non-limiting detailed description and examples.

As used herein, the terms "a/an," "said," "the," "at least one," and "one or more" may be used interchangeably.

To facilitate a clearer understanding of the present invention, certain terms are first defined, and additional definitions are provided throughout the detailed description.

The present invention uses a conventional solid-phase peptide synthesis method to obtain a solid-phase peptide. The safety-catch linker is then activated to form a leaving group, and a reducing agent is used to perform a reduction reaction at the connecting site between the linker and the amino acid residue; simultaneously, the resin is cleaved to obtain a peptidol. This approach is applicable to various amino acids, making it more versatile than the methods described above. In previous solid-phase peptide synthesis, examples of direct use of a reducing agent to react with resin to obtain a peptidol are extremely rare. Therefore, the present invention employs a reducing agent to perform a reductive cleavage reaction, thereby directly obtaining a peptidol without complicated posttreatment and purification procedures.

Specifically, the present invention adopts the safety-catch resin approach, in which 3,4-diaminobenzoic acid (Dbz) resin is activated by isoamyl nitrite to form a benzotriazole (Bt) resin, and then sodium borohydride (NaBH₄) is used as a reducing agent to undergo a reduction reaction, which is fast, requires no complicated purification steps, and a peptidol can be obtained by a simple filtration. After the reaction, hydrolysis and alcoholysis (e.g., ethyl esters) by-products may form. Hydrolysis by-products (e.g., acidic compounds) can be removed by replacing the reducing solvent with dimethylformamide (DMF) to reduce the water content of the solvent. Alcoholysis by-products can be reduced to peptidols by repeating the reduction step. After aspartic acid is reduced, the protective groups are removed under acidic conditions, a step prone to lactonization. Therefore, the step of removing the protective groups in acid requires being performed under an ice bath to suppress lactonization.

Accordingly, the present invention provides a simple and rapid method for preparing a peptidol, which is applicable to common amino acids. Through the design of a linker, the peptide C-terminus can be cleaved from the resin and a peptidol is simultaneously formed by using only a mild reducing agent. In solid-phase peptide synthesis, 4-amino-3-nitrobenzoic acid (ANB) is connected to a Rink amide resin, and the nitro group is reduced to an amino group by using stannous chloride dihydrate and 1,8-diazabicyclo[5.4.0]undec-7-ene (DBU). The linker is converted into 3,4-diaminobenzoic acid (Dbz), and then coupled with the amino acid. After obtaining a target sequence through the solid-phase peptide synthesis, the Dbz resin is converted into benzotriazole (Bt) resin through a reaction with isoamyl nitrite. Considering the swelling of the resin and better solubility of sodium borohydride (NaBH₄) in an alcohol solvent, DCM: EtOH = 3:1 is selected as a solvent system, NaBH₄ is used for conducting reduction reaction, the condition for peptidol synthesis is under an ice bath for 1 hour followed by reaction for 0.5 hours at room temperature to obtain the peptidol.

Accordingly, the present invention provides a method for synthesizing a peptidol, comprising:
(a) reacting a compound of formula (Ia) with a nitrite and acetic acid in a polar solvent to obtain a compound of formula (IIa) having a benzotriazole structure, 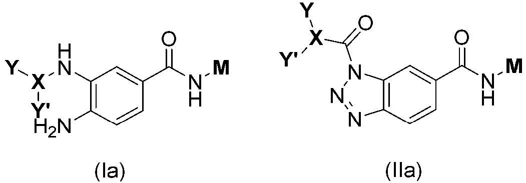
   wherein M is a solid support material;
   X is a peptide;
   Y and Y' are protective groups for protecting N-terminus of the X; and
(b) reacting the compound of formula (IIa) with sodium borohydride in a mixed solution of a polar aprotic solvent and an alcohol solvent for reducing C-terminus of the peptide to obtain a peptidol compound.

In one embodiment, the solid support material comprises a resin.

In another embodiment, the polar solvent comprises dimethylformamide (DMF).

In another embodiment, the nitrite comprises isoamyl nitrite.

In one embodiment, the polar aprotic solvent comprises dichloromethane (DCM) and tetrahydrofuran (THF), and the alcohol solvent comprises methanol, ethanol, propanol, butanol, or the like. In the mixed solution of the polar aprotic solvent and the alcohol solvent, the ratio of the polar aprotic solvent to the alcohol solvent ranges from 10:1 to 1:10, preferably from 5:1 to 1:5, and more preferably from 3:1 to 1:3.

In another more specific embodiment, the reaction condition of the step (b) is an initial ice bath for 1 hour, and then a reaction for 0.5 hour at room temperature.

In another more specific embodiment, the present invention further comprises a step (c) after the step (b), wherein the step (c) comprises removing protective groups on the peptidol compound.

When the molecule of the synthesized peptidol is oversize (e.g., a branched peptide, the efficiency of reductive cleavage is poor and the yield is extremely low), a resin with lower steric hindrance (for example, TentaGel resin) or a linker (6-aminohexanoic acid) should be used to improve the yield.

### BRIEF DESCRIPTION OF THE DRAWINGS

FIG. 1 shows a synthesized peptidol product using NH₂-AQETAol as an example.
FIG. 2 shows an HPLC chromatogram of a synthesized peptidol product using AQETV as an example. The horizontal axis represents retention time, and the vertical axis represents signal intensity.
FIG. 3 shows an HPLC chromatogram of a synthesized peptidol product with a high proportion of ethyl ester by-products. The horizontal axis represents retention time, and the vertical axis represents signal intensity.
FIG. 4 shows an HPLC chromatogram of a synthesized peptidol product with a high proportion of hydrolysis by-products. The horizontal axis represents retention time, and the vertical axis represents signal intensity.
FIG. 5 shows an HPLC chromatogram of a synthesized peptidol product suitable for aspartic acid and asparagine. The horizontal axis represents retention time, and the vertical axis represents signal intensity.
FIG. 6 shows an HPLC chromatogram of a synthesized peptidol product suitable for branched peptide synthesis. The horizontal axis represents retention time, and the vertical axis represents signal intensity.
FIG. 7 shows an HPLC chromatogram of a synthesized peptidol product suitable for peptaibol synthesis. The horizontal axis represents retention time, and the vertical axis represents signal intensity.
FIG. 8 shows an HPLC chromatogram of a synthesized peptidol product suitable for small-molecule library synthesis. The horizontal axis represents retention time, and the vertical axis represents signal intensity.

### EXAMPLES

The following examples are non-limiting and are provided merely to illustrate various aspects and features of the present invention.

The present invention establishes several C-terminal reduction methods. Method 1: The mixture of dichloromethane: alcohol was used as a solvent system for performing C-terminal reduction reaction, which was suitable for synthesizing a peptidol without severe hydrolysis or ethanolysis. When severe hydrolysis or ethanolysis occurred, Methods 2 or 3 should be adopted for synthesis instead. Method 2: The mixture of dichloromethane: alcohol was used as a solvent system for performing a second C-terminal reduction reaction. This method could reduce ethanolysis by-products back to peptidols. But peptidols with severe hydrolysis could not be corrected by this method, and Method 3 should be adopted. Method 3: Dimethylformamide (DMF) was used as a solvent system for performing C-terminal reduction reaction. This method effectively minimized the formation of hydrolysis by-products, thereby improving the efficiency of peptidol synthesis. Method 4: The subsequent acidic cleavage of side-chain protective groups was changed to be performed in an ice bath for 1 hour. This method could reduce the proportion of lactone derivatives formed by C-terminal hydroxyl group which attacks side-chain functional groups such as those of aspartic acid. However, this method could not completely prevent formation of lactone derivatives. The C-terminal reduction method was successfully applied to twenty different amino acids.

0.3333 g (0.1 mmol) of Rink amide resin (loading: 0.3 mmol/g) was added to DMF (4mL), soaked with shaking for 1 hour. 20% Piperidine in DMF was added and reacted for 10 minutes to remove Fmoc group, dried, and repeated the procedure once. Washed alternately with DCM and DMF three times. ANB (0.0728 g, 0.4 mmol, 4 eq) and hexafluorophosphate benzotriazole tetramethyl uranium (HBTU) (0.1516 g, 0.4 mmol, 4 eq) were added, then DMF (5% NMM, 3 mL) was added, and the reaction was allowed to proceed for 2 hours. Washed sequentially with DCM and DMF three times. The resin was soaked in DMF (4 mL), then tin(II) chloride dihydrate (2.256 g, 98%, 2.5 M, 11.89 mmol, 119 eq) and DBU (300 µL, 2 mmol, 20 eq) were added, and the mixture was reacted for 6 hours. Washed sequentially with DCM and DMF five times. The first amino acid (4 eq) and benzotriazol-1-yloxytripyrrolidinophosphonium hexafluorophosphate (PyBop) (0.2080 g, 0.4 mmol, 4 eq) were added, reacted in DMF (5% NMM (3 mL) for 2 hours, dried, and repeated one more time. Washed sequentially with DCM and DMF three times.

After a target solid-phase peptide was obtained via SPPS, peptidols were synthesized using four different methods. Method 1 was used first for synthesis; when the proportion of byproducts was high, Methods 2, 3, or 4 were used for synthesis.

### Example 1

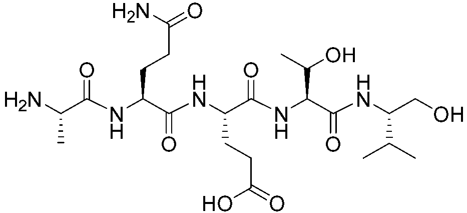

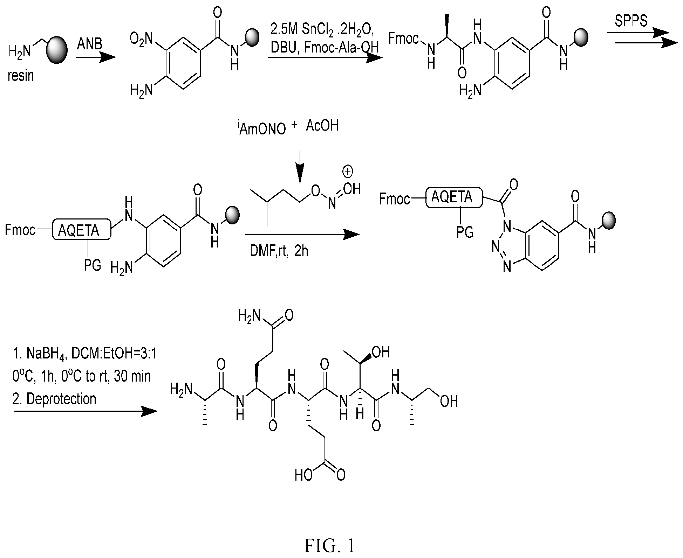

Fmoc-AQETV-Dbz-Rink amide resin (0.05 mmol) was added to DMF (4 mL), and soaked with shaking for 1 hour. Isoamyl nitrite (60 eq, 400 µL) was mixed with acetic acid (1.8 eq, 5.14 µL), mixed for 30 minutes, added to the resin, and activated for 2 hours. Washed sequentially with DCM and DMF five times. The resin was transferred to a 25 mL round-bottom flask, DCM: EtOH = 3:1 (4 mL) was added, placed in an ice bath, NaBH₄ (5 eq, 9.4 mg) was added, reacted in the ice bath for 1 hour; after the ice bath was removed, reacted for an additional 30 minutes, and the filtrate was collected, as shown in FIG. 1. Organic layers were washed twice with saturated brine, dried with anhydrous MgSO₄, filtered, concentrated to dry under reduced pressure. 20% piperidine in DMF was added, and reacted for 30 minutes to remove Fmoc, and dried. TFA: TIS: H₂O = 95:2.5:2.5 (v/v/v) (2 mL) was added, and reacted for 30 minutes. Iced diethyl ether was added to precipitate, centrifuged, and lyophilized.

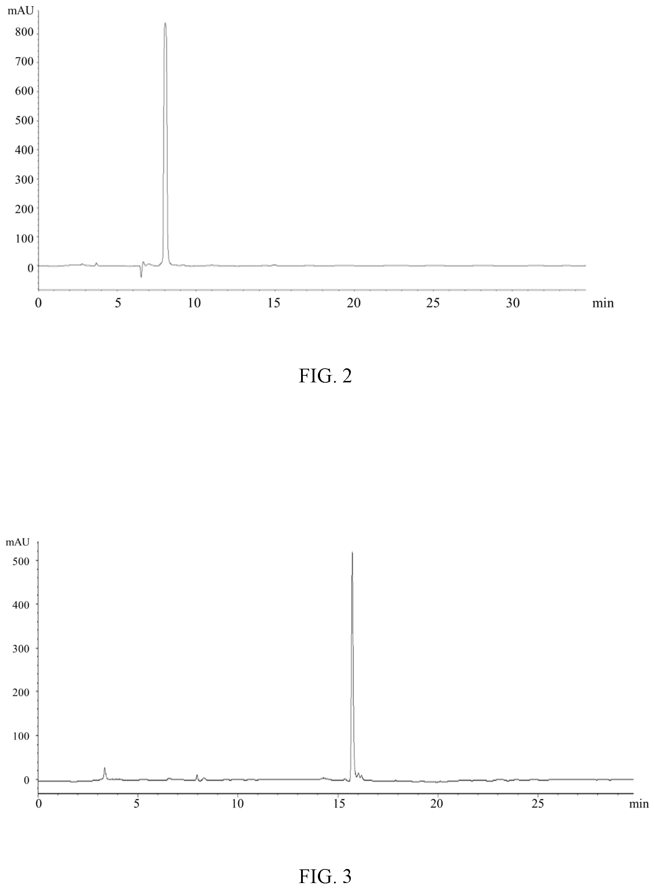

FIG. 2 showed an HPLC chromatogram of a synthesized peptidol product of this embodiment. The horizontal axis represented retention time, and the vertical axis represented signal intensity.

### Example 2: Method suitable for high proportion of ethyl ester by-products

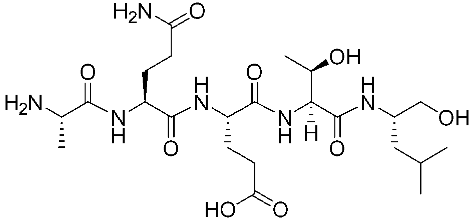

Fmoc-AQETL-Dbz-Rink amide resin (0.05 mmol) was added to DMF (4 mL), and soaked with shaking for 1 hour. Isoamyl nitrite (60 eq, 400 µL) was mixed with acetic acid (1.8 eq, 5.14 µL) for 30 minutes, then added to the resin, which was then activated for 2 hours. Washed sequentially with DCM and DMF five times. The resin was transferred to a 25 mL round-bottom flask, DCM: EtOH = 3:1 (4 mL) was added, placed in an ice bath, NaBH₄ (5 eq, 9.4 mg) was added, reacted in the ice bath for 1 hour, after the ice bath was removed, reacted for an additional 30 minutes, and the filtrate was collected. After being dried, DCM: EtOH = 3:1 (4 mL) was added again, placed in an ice bath, NaBH₄ (5 eq, 9.4 mg) was added, reacted in the ice bath for 1 hour, after the ice bath was removed, reacted for an additional 30 minutes, and the filtrate was collected. The organic layers were washed twice with saturated brine, dried with anhydrous MgSO₄, filtered, and concentrated to dry under reduced pressure. 20% piperidine in DMF was added, and reacted for 30 minutes to remove Fmoc, and dried. TFA: TIS: H₂O = 95:2.5:2.5 (v/v/v) (2 mL) was added, and reacted for 30 minutes. Iced diethyl ether was added to precipitate, the mixture was centrifuged, and then lyophilized.

FIG. 3 showed an HPLC chromatogram of a synthesized peptidol product with a high proportion of ethyl ester by-products. The horizontal axis represented retention time, and the vertical axis represented signal intensity.

### Example 3: Method suitable for high proportion of hydrolysis by-products

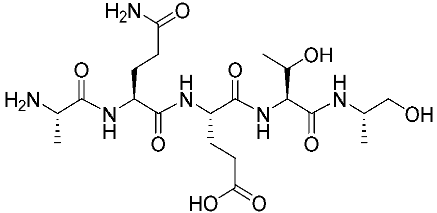

Fmoc-AQETA-Dbz-Rink amide resin (0.05 mmol) was added to DMF (4 mL), and soaked with shaking for 1 hour. Isoamyl nitrite (60 eq, 400 µL) was mixed with acetic acid (1.8 eq, 5.14 µL) for 30 minutes, then added to the resin and activated for 2 hours. Washed sequentially with DCM and DMF five times. The resin was transferred to a 25 mL round-bottom flask, placed in an ice bath, DMF (4 mL) was added, NaBH₄ (5 eq, 0.0094 g) was added, the resin turned orange-red in color, reacted for 1 hour in the ice bath, after the ice bath was removed, reacted for additional 30 minutes, and the filtrate was collected. Organic layers were washed twice with saturated brine, dried with anhydrous MgSO₄, filtered, and concentrated to dry under reduced pressure. 20% piperidine in DMF was added, reacted for 30 minutes, Fmoc was removed, and the product was dried. TFA: TIS: H₂O (95:2.5:2.5, v/v/v) (2 mL) was added, and the mixture was reacted for 30 minutes. Iced diethyl ether was added to precipitate, centrifuged, and lyophilized.

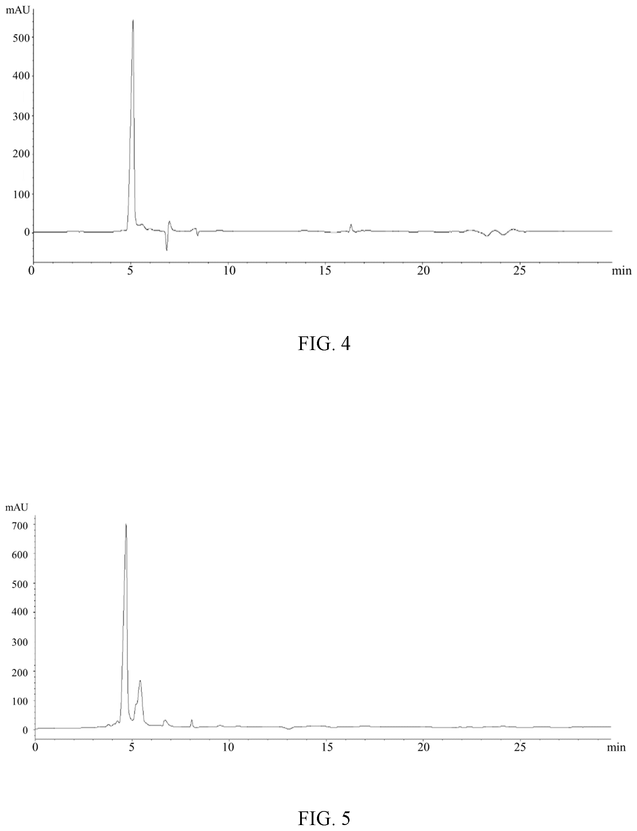

FIG. 4 showed an HPLC chromatogram of a synthesized peptidol with high proportion of hydrolysis by-products. The horizontal axis represented retention time, and the vertical axis represented signal intensity.

### Example 4: Method suitable for aspartic acid

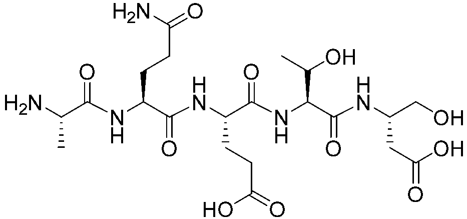

Fmoc-AQETD-Dbz-Rink amide resin (0.05 mmol) was added to DMF (4 mL), and soaked with shaking for 1 hour. Isoamyl nitrite (60 eq, 400 µL) was mixed with acetic acid (1.8 eq, 5.14 µL) for 30 minutes, then added to the resin and activated for 2 hours. Washed sequentially with DCM and DMF five times. The resin was transferred to a 25 mL round-bottom flask, placed in an ice bath, DCM: EtOH (3:1, 4 mL) was added, NaBH₄ (5 eq, 0.0094 g) was added, reacted in the ice bath for 1 hour, after the ice bath was removed, reacted for an additional 30 minutes, and the filtrate was collected. Organic layers were washed twice with saturated brine, dried with anhydrous MgSO₄, filtered, and concentrated to dry under reduced pressure. 20% piperidine in DMF was added, and stirred for 30 minutes to remove Fmoc, and dried. TFA: TIS: H₂O (95:2.5:2.5, v/v/v) (2 mL) was added, and reacted in an ice bath for 1 hour. Iced diethyl ether was added to precipitate, centrifuged and lyophilized.

FIG. 5 showed an HPLC chromatogram of a synthesized peptidol product of aspartic acid or asparagine, and the one with higher signal was the desired product. The horizontal axis represented retention time, and the vertical axis represented signal intensity.

### Example 5: Method suitable for branched peptide synthesis

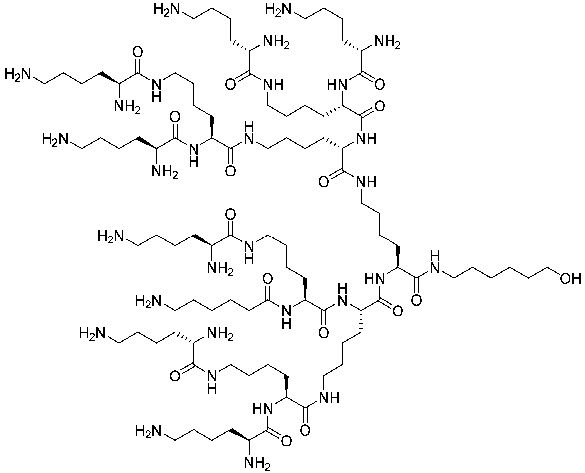

0.4166 g (0.1 mmol) TentaGel resin (loading: 0.24 mmol/g) was added to DMF (4 mL), soaked with shaking for 1 hour. 20% piperidine in DMF was added, and reacted for 10 minutes to remove Fmoc, dried, and the procedure was repeated once. Washed sequentially with DCM and DMF three times. Fmoc-6-aminocarbamoyl-Dbz (0.1948 g, 0.4 mmol, 4 eq) and HBTU (0.1516 g, 0.4 mmol, 4 eq) were added, DMF (5% NMM, 3 mL) was added, and the reaction was allowed to proceed for 2 hours. Washed sequentially with DCM and DMF three times. 20% piperidine in DMF was added, and reacted for 10 minutes to remove Fmoc, dried, and the procedure was repeated once. Washed sequentially with DCM and DMF three times. Fmoc-L-Lys(Fmoc)-OH (0.1040 g, 0.175 mmol, 3.5 eq) and HBTU (0.1326 g, 0.175 mmol, 3.5 eq) were added, DMF (5% NMM, 2 mL) was added, and the reaction was allowed to proceed for 2 hours. Washed sequentially with DCM and DMF three times. 20% piperidine was added, dissolved in DMF, and reacted for 10 minutes to remove Fmoc, suctioned to dry, the procedure was repeated once, and washed sequentially with DCM and DMF three times. Fmoc-L-Lys(Fmoc)-OH (0.2060 g, 0.35 mmol, 3.5 eq) and HBTU (0.2652 g, 0.35 mmol, 3.5 eq) were added, DMF (5% NMM, 2 mL) was added, and reacted for 1 hour. Washed sequentially with DCM and DMF three times. 20% piperidine was added, dissolved in DMF, and reacted for 10 minutes to remove Fmoc, dried, and the procedure was repeated once. Washed sequentially with DCM and DMF three times. Fmoc-L-Lys(Fmoc)-OH (0.4130 g, 0.7 mmol, 3.5 eq) and HBTU (0.5304 g, 0.7 mmol, 3.5 eq) were added, DMF (5% NMM, 2 mL) was added, and reacted for 2 hours. Washed sequentially with DCM and DMF three times. 20% piperidine was added, dissolved in DMF, and reacted for 10 minutes to remove Fmoc, dried, and the procedure was repeated once. Washed sequentially with DCM and DMF three times. Boc-L-Lys(Boc)-OH (0.8260 g, 1.4 mmol, 3.5 eq) and HBTU (1.0608 g, 1.4 mmol, 3.5 eq) were added, DMF (5% NMM, 4 mL) was added, and reacted for 2 hours. Washed sequentially with DCM and DMF three times to obtain Boc-K8(Boc)K4K2K-Dbz-TentaGel resin.

Boc-K₈(Boc)K₄K₂K-Dbz-TentaGel resin (0.05 mmol) was added to DMF (4 mL), and soaked with shaking for 1 hour. Isoamyl nitrite (60 eq, 400 µL) was mixed with acetic acid (1.8 eq, 5.14 µL) for 30 minutes, then added to the resin and activated for 2 hours. Washed sequentially with DCM and DMF five times. The resin was transferred to a 25 mL round-bottom flask, DCM: EtOH = 3:1 (4 mL) was added, placed in an ice bath, NaBH₄ (5 eq, 9.4 mg) was added, reacted in the ice bath for 1 hour, after the ice bath was removed, reacted for an additional 30 minutes, and the filtrate was collected. Organic layers were washed twice with saturated brine, dried with anhydrous MgSO₄, filtered, and concentrated to dry under reduced pressure. TFA: TIS: H₂O (95:2.5:2.5, v/v/v) (2 mL) was added, and reacted for 30 minutes. Iced diethyl ether was added to precipitate, centrifuged and lyophilized.

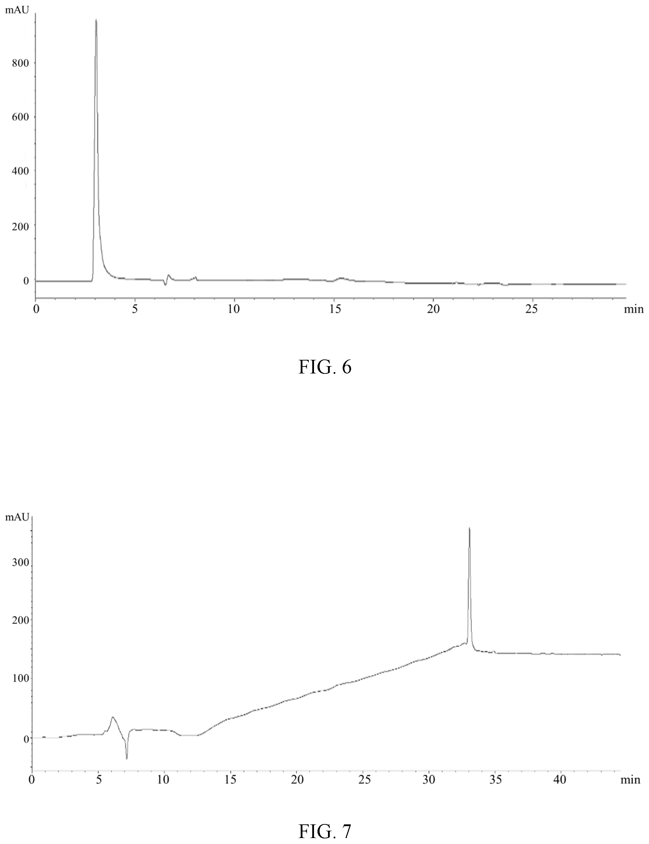

FIG. 6 showed an HPLC chromatogram of a synthesized peptide product synthesized from branched peptides. The horizontal axis represented retention time, and the vertical axis represented signal intensity.

### Example 6: Method suitable for peptaibol synthesis

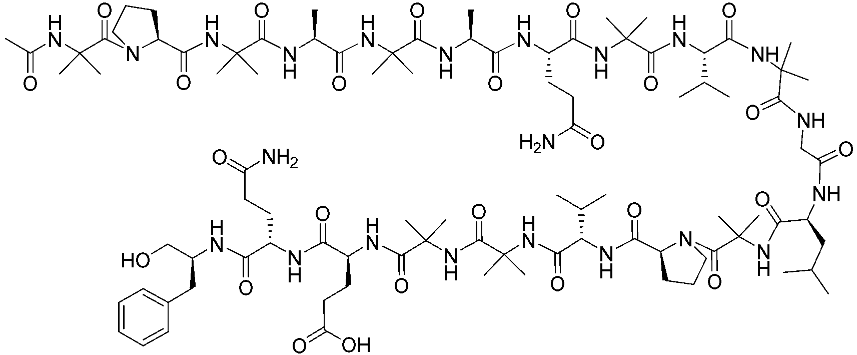

0.3333 g (0.1 mmol) of Rink amide resin (loading: 0.3 mmol/g**)** was added to DMF (4 mL)**,** and soaked with shaking for 1 hour. 20% piperidine in DMF was added, and reacted for 10 minutes to remove the Fmoc group, dried, and the procedure was repeated once. Washed sequentially with DCM and DMF three times. ANB (0.0728 g, 0.4 mmol, 4 eq) and HBTU (0.1516 g, 0.4 mmol, 4 eq) were added, DMF (3 mL, containing 5% NMM) was added, and the reaction was allowed to proceed for 2 hours. Washed sequentially with DCM and DMF three times. The resin was soaked in DMF (4 mL), tin(II) chloride dihydrate (2.256 g, 98%, 2.5 M, 11.89 mmol, 119 eq), and DBU (300 µL, 2 mmol, 20 eq) were added, and reacted for 6 hours. Washed sequentially with DCM and DMF five times. Fmoc-Phe-OH (155 mg, 0.4 mmol, 4 eq) and PyBop (0.2080 g, 0.4 mmol, 4 eq) were added, reacted in DMF (3 mL, containing 5% NMM) for 2 hours, dried, and the procedure was repeated once. Washed sequentially with DCM and DMF three times. Subsequently, a target solid-phase peptide was synthesized through the SPPS procedure. Fmoc-amino acid was dissolved in DMF (0.2 M), an equal amount of Oxyma (0.5 M in DMF) was added and dissolved in DMF (0.5 M), and two times of DIC (1.0 M in DMF) was added and dissolved in DMF (1.0 M). 20% piperidine in DMF was added to DMF (4 mL) to remove Fmoc protective groups twice, 10 minutes each time. Upon completion of the reactions, the resin was washed sequentially with DCM and DMF three times. Unless otherwise specified, the coupling time was 1 hour. The order and equivalents of Fmoc-amino acids to be added are detailed below. Fmoc-L-Gln(Trt)-OH (122 mg, 0.2 mmol, 2 eq), Fmoc-L-Glu(OtBu)-OH (85 mg, 0.2 mmol, 2 eq), Fmoc-Aib-OH (131 mg, 0.4 mmol, 4 eq) were coupled for 2 hours, Fmoc-Aib-OH (131 mg, 0.4 mmol, 4 eq)was coupled for 3 hours, Fmoc-L-Val-OH (136 mg, 0.4 mmol, 4 eq) was coupled for 2 hours, Fmoc-L-Pro-OH (136 mg, 0.4 mmol, 4 eq) and Fmoc-Aib-OH (131 mg, 0.4 mmol, 4 eq) were coupled for 2 hours and repeated twice, Fmoc-L-Leu-OH (142 mg, 0.4 mmol, 4 eq) and Fmoc-L-Gly-OH (120 mg, 0.4 mmol, 4 eq) were coupled for 1.5 hours, Fmoc-Aib-OH (131 mg, 0.4 mmol, 4 eq), Fmoc-L-Val-OH (136 mg, 0.4 mmol, 4 eq) and Fmoc-Aib-OH (131 mg, 0.4 mmol, 4 eq) were coupled for 4 hours, Fmoc-L-Gln(Trt)-OH (245 mg, 0.4 mmol, 4 eq) and Fmoc-L-Ala-OH (125 mg, 0.4 mmol, 4 eq) were coupled for 2 hours, Fmoc-Aib-OH (131 mg, 0.4 mmol, 4 eq), Fmoc-L-Ala-OH (62 mg, 0.2 mmol, 2 eq), and Fmoc-Aib-OH (131 mg, 0.4 mmol, 4 eq) were coupled for 2 hours, Fmoc-L-Pro-OH (136 mg, 0.4 mmol, 4 eq) and Fmoc-Aib-OH (131 mg, 0.4 mmol, 4 eq) were coupled with ultrasonic assistance, 10 minutes each time for two times. Finally, acetic anhydride (20 µL) in 5% NMM was dissolved in 5% DMF (2 mL) and reacted for 30 minutes.

Ac-UPUAUAQUVUGLUPVUUEQF-Dbz-Rink amide resin (0.05 mmol) was added in DMF (4 mL), soaked with shaking for 1 hour. Isoamyl nitrite (60 eq, 400 µL) was mixed with acetic acid (1.8 eq, 5.14 µL) for 30 minutes, then added to the resin and activated for 2 hours. Washed sequentially with DCM and DMF five times. The resin was transferred to a 25 mL round-bottom flask, DCM: EtOH = 3:1 (4 mL) was added, placed in ice bath, NaBH₄ (5 eq, 9.4 mg) was added, reacted in the ice bath for 1 hour, after the ice bath was removed, reacted for an additional 30 minutes, and filtrate was collected. Organic layers were washed twice with saturated brine, dried with anhydrous MgSO₄, filtered, and concentrated to dry under reduced pressure. TFA: TIS: H₂O = 95:2.5:2.5 (v/v/v, 2 mL) was added, and reacted for 30 minutes. Iced ethyl ether was added to precipitate, filtered, centrifuged, and lyophilized.

FIG. 7 showed an HPLC chromatogram of a synthesized peptidol product from peptaibols synthesis. The horizontal axis represented the retention time, and the vertical axis represented the signal intensity.

### Example 7: for small molecule library synthesis

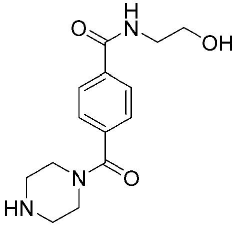

0.3333 g (0.1 mmol) of Rink amide resin (loading: 0.3 mmol/g) was added to DMF (4 mL), and soaked with shaking for 1 hour. 20% piperidine in DMF was added, and the mixture was reacted for 10 minutes to remove the Fmoc, dried, and the procedure was repeated once. Washed sequentially with DCM and DMF three times. ANB (0.0728 g, 0.4 mmol, 4 eq) and HBTU (0.1516 g, 0.4 mmol, 4 eq) were added to DMF (5% NMM, 3 mL), and reacted for 2 hours. Washed sequentially with DCM and DMF three times. The resin was soaked in DMF (4 mL), tin(II) chloride dihydrate (2.256 g, 98%, 2.5 M, 11.89 mmol, 119 eq) and DBU (300 µL, 2 mmol, 20 eq) were added, and reacted for 6 hours. Washed sequentially with DCM and DMF five times. Fmoc-Gly-OH (0.1189 g, 0.4 mmol, 4 eq) and PyBOP (0.2080 g, 0.4 mmol, 4 eq) were added, reacted in DMF (5% NMM, 3 mL) for 2 hours, dried, and the procedure was repeated once. Washed sequentially with DCM and DMF three times. Subsequently, a target solid-phase peptide was synthesized through the SPPS procedure, the desired coupling reagents were dissolved in DMF (0.2 M), and an equal amount of Oxyma in DMF (0.5 M) and two times of DIC in DMF (1.0M) were added. 20% piperidine in 4 mL of DMF was added twice and reacted for 10 minutes each time to remove the Fmoc groups. Upon completion of the reactions, the resin was washed sequentially with DCM and DMF three times. The coupling time was 2 hours. The order and equivalents of the coupling reagent to be added are described in detail below. Terephthalic acid (0.0332 g, 0.2 mmol, 2 eq) and Boc-piperazine (0.0373 g, 0.2 mmol, 2 eq). Boc-piperazine-TPA-G-Dbz-Rink amide resin (0.05 mmol) was added to DMF (4 mL), and the mixture was shaken for 1 hour. Isoamyl nitrite (60 eq, 400 µL) was mixed with acetic acid (1.8 eq, 5.14 µL) for 30 minutes, then added to the resin and activated for 2 hours. Washed sequentially with DCM and DMF five times. The resin was transferred to a 25 mL round-bottom flask, DCM: EtOH = 3:1 (4 mL) was added, placed in an ice bath, NaBH₄ (5 eq, 9.4 mg) was added, reacted in the ice bath for 1 hour, after the ice bath was removed, reacted for an additional 30 minutes, and the filtrate was collected. Organic layers were washed twice with saturated brine, dried with anhydrous MgSO₄, filtered, and concentrated to dry under reduced pressure. 20% TFA in DCM (2 mL) was added and reacted for 30 minutes, dried, and iced diethyl ether was added to precipitate, centrifuged, and lyophilized.

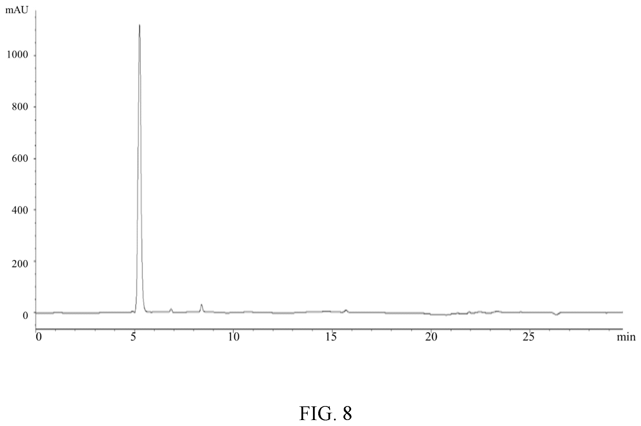

FIG. 8 showed an HPLC chromatogram of a synthesized peptidol product synthesized from a small-molecule library. The horizontal axis represented retention time, and the vertical axis represented signal intensity.

### Example 8: Optimization of Peptide C-terminal reduction reaction

Fmoc-AQETA-Dbz-Rink amide was used to optimize the conditions. In the following discussion about product ratios in reactions, unless otherwise specified, the sum of the integrated peak areas of the main products recorded in SIM mode of the mass spectrum was used as the denominator, and the integrated peak area of the target product was used as the numerator to calculate the proportions in the main products. First, an appropriate reaction solvent needed to be selected, taking into account resin swelling and the better solubility of sodium borohydride in alcohols; therefore, THF: CH₃OH was used to start solvent system tests. When THF: CH₃OH = 1:3, 93% of NH₂-AQETA-COOCH₃ was found in crude products, indicating that methanolysis was the dominant reaction. Furthermore, during the reaction, most of the resins adhered to the walls of the reaction flask, thereby impairing the reaction. It was speculated that the increased proportion of methanol in the solvent raised the polarity of the environment. As a hydrophobic structure, the resin tended to aggregate in the solvent and could not swell effectively, resulting in reduced yield. Therefore, the solvent system was changed to THF: CH₃OH = 3:1, and the proportion of peptidol reached 86%; however, 3% NH₂-AQETA-COOCH₃ and 11% hydrolysis by-products were still observed. To avoid methanolysis, the solvent was changed to DCM: EtOH, and only the main product NH₂-AQETAol was formed. The ratios of the two solvents were then adjusted. However, under the same conditions, the by-product signal gradually increased with the ethanol proportion. Based on molecular weight, it was inferred to be an ethanolysis product NH₂-AQETA-COOCH₂CH₃. As the proportion of DCM increased, the proportion of peptidol also increased, while the proportions of ethanolysis and hydrolysis decreased. Even when the ratio of DCM: EtOH was 1:3, the proportion of ethanolysis was more significant than that without methanolysis. Therefore, DCM: EtOH = 3:1 was selected as the solvent system for subsequent reactions, as shown in Table 1.

Table 1. Product ratios of NH₂-AQETAol under different reduction conditions.

| Solvent | Yield (%) | | | |
|---|---|---|---|---|
| | Peptidol | Acid | Ethyl ester | Methyl ester |
| DCM: EtOH (3:1) | 85 | 11 | 4 | ND |
| DCM: EtOH (1:1) | 77 | 5 | 18 | ND |
| DCM: EtOH (1:3) | 51 | 4 | 45 | ND |
| THF: CH₃OH (1:3) | 5 | 2 | ND | 93 |
| THF: CH₃OH (3:1) | 86 | 11 | ND | 3 |

Product ratio: The main product m/z was set in SIM mode of the mass spectrum of crude products; the sum of the integrated peak areas of the main product m/z was used as the denominator to calculate the proportion in the main product; ND: not detected.

Although the invention has been described and exemplified in sufficient detail to enable those skilled in the art to make and use it, various alternatives, modifications, and improvements will be apparent without departing from the spirit and scope of the invention

Those skilled in the art will readily appreciate that the invention is well-suited to achieve the above-stated objectives and advantages, including those inherent therein. The processes and methods described for producing them represent preferred embodiments, are exemplary, and do not limit the scope of the invention. Those of ordinary skill in the art will recognize possible modifications and other uses, which are included within the spirit of the invention and are defined by the scope of the claims.

## Claims

1. A method for synthesizing a peptidol, comprising:
(a) reacting a compound of formula (Ia) with a nitrite and acetic acid in a polar solvent to obtain a compound of formula (IIa) having a benzotriazole structure, 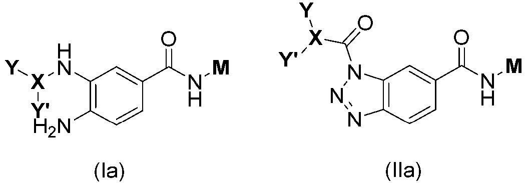
wherein M is a solid-phase support material;
X is a peptide;
Y and Y' are protective groups for protecting N-terminus of the X; and
(b) reacting the compound of formula (IIa) with sodium borohydride in a mixed solution of a polar aprotic solvent and an alcohol solvent for reducing C-terminus of the peptide to obtain a peptidol compound.

2. The method of claim 1, wherein the solid support material comprises a resin.

3. The method of claim 1, wherein the polar solvent comprises dimethylformamide (DMF), and the nitrite comprises isoamyl nitrite.

4. The method of claim 1, wherein the polar aprotic solvent comprises dichloromethane, and the alcohol solvent comprises ethanol.

5. The method of claim 1, wherein the reaction condition of the step (b) is an initial ice bath for 1 hour, and then a reaction for 0.5 hour at room temperature.

6. The method of claim 1, which further comprises a step (c) after the step (b), and the step (c) comprises removing protective groups on the peptidol compound.

7. The method of claim 1, wherein the step (b) produces hydrolysis by-products or ethanolysis by-products.

8. The method of claim 7, wherein the hydrolysis by-products are removed by substituting the reduction solvent with dimethylformamide (DMF) and reducing water content of the solvent.

9. The method of claim 7, wherein the ethanolysis by-products are reduced to peptidols by repeating the reduction of the ethanolysis by-products.

10. The method of claim 1, wherein when the peptide is aspartic acid, after reduction of the aspartic acid, the step of removing protective groups in an acid is performed in an ice bath to suppress lactonization.
